(19) Europäisches Patentamt
European Patent Office
Office européen des brevets

(11) Veröffentlichungsnummer: **0 284 893**
**A2**

(12) # EUROPÄISCHE PATENTANMELDUNG

(21) Anmeldenummer: **88104164.4**

(22) Anmeldetag: **16.03.88**

(51) Int. Cl.[4]: **C07D 513/14** , A61K 31/425 , //(C07D513/14,333:00,277:00, 235:00)

Patentansprüche für folgende Vertragsstaaten: ES + GR.

(30) Priorität: **03.04.87 AT 821/87**

(43) Veröffentlichungstag der Anmeldung: **05.10.88 Patentblatt 88/40**

(84) Benannte Vertragsstaaten: **AT BE CH DE ES FR GB GR IT LI LU NL SE**

(71) Anmelder: **CL PHARMA AKTIENGESELLSCHAFT**
**St. Peter-Strasse 25**
**A-4021 Linz(AT)**

(72) Erfinder: **Binder, Dieter, Dr.**
**Sieveringerstrasse 207**
**A-1190 Wien(AT)**
Erfinder: **Rovenszky, Franz, Dr. Dipl.-Ing.**
**Lagerhausstrasse 5/8**
**A-2460 Bruck a.d. Leitha(AT)**
Erfinder: **Ferber, Hubert Peter, Dr.**
**Ahornweg 24**
**A-4021 Ansfelden(AT)**

(74) Vertreter: **Kunz, Ekkehard, Dr.**
**Chemie Holding AG Patentwesen St. Peter-Strasse 25**
**A-4021 Linz(AT)**

(54) **Neue Thieno-imidazo(2,1-b)thiazol-Derivate, ein Verfahren zu ihrer Herstellung und diese enthaltende pharmazeutische Präparate.**

(57) Die Erfindung betrifft neue Thienoimidazo(2,1-b)thiazolderivate der Formel

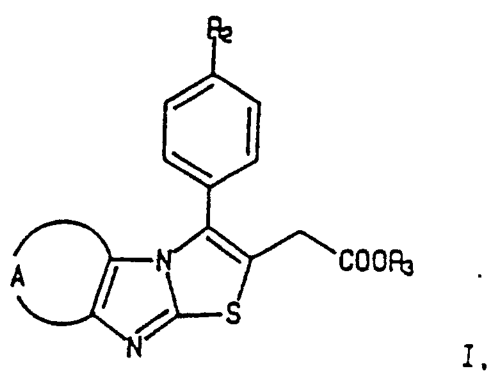

I,

in der
A zusammen mit den beiden Kohlenstoff-Atomen des Imidazolrings eine Gruppe der Formel

EP 0 284 893 A2

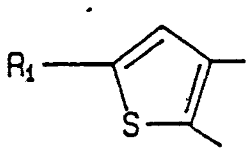

oder

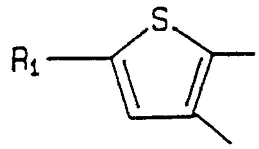

IIa IIb

bildet,

$R_1$ Wasserstoff, $C_1$-$C_4$ Alkyl, Halogen oder $CF_3$,

$R_2$ Wasserstoff, Halogen oder $CF_3$ und

$R_3$ Wasserstoff oder $C_1$-$C_4$ Alkyl bedeuten und, für den Fall, daß $R_3$ Wasserstoff bedeutet, ihre pharmazeutisch verwendbaren Salze, ein Verfahren zur Herstellung dieser Verbindungen und deren Anwendung zur Behandlung von Krebs oder rheumatischer Arthritis, hervorgerufen durch ein defektes Immunsystem.

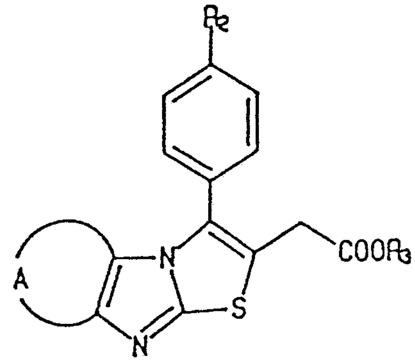

I.

# Neue Thieno-imidazo(2,1-b)thiazolderivate, ein Verfahren zu ihrer Herstellung und diese enthaltende pharmazeutische Präparate

Die Erfindung betrifft neue Thieno-imidazo(2,1-b)thiazolderivate, ein Verfahren zu ihrer Herstellung und ihre Verwendung in Medikamenten zur Stimulierung des Immunsystems.

Gegenstand der Erfindung sind daher neue Verbindungen der Formel I des Formelblattes, in der A zusammen mit den zwei Kohlenstoffatomen des Imidazolrings eine Gruppe IIa oder IIb des Formelblattes bildet, $R_1$ Wasserstoff, $C_1$ -$C_4$ Alkyl, Halogen oder Trifluormethyl, $R_2$ Wasserstoff, Halogen oder Trifluormethyl und $R_3$ Wasserstoff oder $C_1$ -$C_4$ Alkyl bedeuten und, für den Fall, daß $R_3$ Wasserstoff bedeutet, ihre pharmazeutisch verwendbaren Salze.

Der in dieser Beschreibung verwendete Ausdruck "$C_1$ -$C_4$ Alkyl" bezeichnet geradkettige oder verzweigte gesättigte Kohlenwasserstoffreste mit 1 bis 4 Kohlenstoffatomen, wie z.B. Methyl, Ethyl, Propyl, Isopropyl, Butyl, Isobutyl, tert.Butyl. Der Ausdruck "Halogen" bezeichnet Chlor, Brom oder Fluor.

Eine bevorzugte Klasse der Verbindungen der Formel I ist jene, in der $R_1$ Wasserstoff, $R_2$ Chlor und $R_3$ Wasserstoff oder Methyl bedeutet.

Besonders bevorzugte Einzelverbindungen sind:

5-(4-Chlorphenyl)-thieno(2',3'-4,5)imidazo(2,1-b)thiazol-6-essigsäuremethylester
7-(4-Chlorphenyl)-thieno(3',2'-4,5)imidazo(2,1-b)thiazol-6-essigsäuremethylester
5-(4-Chlorphenyl)-thieno(2',3'-4,5)imidazo(2,1-b)thiazol-6-essigsäure
7-(4-Chlorphenyl)-thieno(3',2'-4,5)imidazo(2,1-b)thiazol-6-essigsäure.

Die Thieno-imidazo(2,1-b)thiazolderivate der Formel I und deren Salze werden erfindungsgemäß dadurch hergestellt, daß man

a) eine Verbindung der Formel III des Formelblattes,

in der $R_1$ und $R_2$ die obige Bedeutung haben und $R_3$ $C_1$ -$C_4$ Alkyl bedeutet, in Gegenwart von wasserentziehenden Reagenzien zu einem Anellierungsisomerengemisch der Formel I, in der A die Gruppen der Formeln IIa und IIb bedeutet und $R_1$, $R_2$ und $R_3$ die oben genannte Bedeutung haben, cyclisiert und das Isomerengemisch trennt, worauf man

b) gegebenenfalls eine so erhaltene Verbindung der Formel I, in der $R_3$ $C_1$ -$C_4$ Alkyl bedeutet, zu Verbindungen der Formel I, worin $R_3$ Wasserstoff bedeutet, alkalisch verseift und

c) erwünschtenfalls eine im Verfahrensschritt b) erhaltene freie Säure der Formel I, in der $R_3$ Wasserstoff bedeutet, mit anorganischen oder organischen Basen in ein pharmazeutisch verträgliches Salz überführt.

Als wasserentziehende Reagentien bei der Cyclisierung von Verbindungen der Formel III können alle üblicherweise verwendeten wasserentziehenden Mittel verwendet werden. Vorzugsweise kommen Polyphosphorsäure oder Phosphoroxychlorid in Frage, die zugleich als Lösungsmittel verwendet werden können. Die Cyclisierungstemperatur soll ca. 60 °C bis 110 °C betragen. Bei Phosphoroxychlorid cyclisiert man am besten bei Rückflußtemperatur. Die Reaktionszeit beträgt, abhängig von der Temperatur und dem Cyclisierungsmittel, etwa zwischen 10 Minuten und 4 Stunden.

Das so erhaltene Anellierungsisomerengemisch, bestehend aus den Estern der Formel I mit der Bedeutung A = IIa und IIb, kann nach allen üblichen Methoden der Isomerentrennung wie beispielsweise Umkristallisation, Säulenchromatographie, Verteilungschromatographie, Extraktion u.ä. getrennt werden. Die Ester der Formel I mit der Bedeutung A = IIa werden vorzugsweise durch mehrmaliges Umkristallisieren aus Methanol isomerenfrei erhalten. Aus den vereinigten Mutterlaugen können dann die Ester der Formel I mit der Bedeutung A = IIb durch Säulenchromatographie auf Kieselgel isomerenfrei erhalten werden, wobei sich Elutionsgemische, in denen sich das Estergemisch gut löst, wie z.B. Gemische aus Methylenchlorid, Chloroform, Benzol oder Toluol einerseits und Ether andererseits eignen. Die Ester der Formel I mit A = IIb werden so als erstes eluiert.

Gegebenenfalls können die Ester der Formel I durch Kochen mit Basen, vorzugsweise mit äquivalenten Mengen Alkalihydroxidlöslungen und unter Zusatz eines Lösungsvermittlers wie z.B. Methanol oder Ethanol, in fast quantitativer Ausbeute zu Verbindungen der Formel I, in der $R_3$ Wasserstoff bedeutet, verseift werden.

Die bei der Umsetzung in Verfahrensschritt b) erhaltenen Verbindungen der Formel I, welche eine freie Carboxylgruppe haben, können auf übliche Weise mit anorganischen oder organischen Basen in ihre pharmazeutisch verwendbaren Salze übergeführt werden. Die Salzbildung kann beispielsweise durchgeführt werden, indem man die genannten Verbindungen der Formel I, in der $R_3$ = H bedeutet, in einem

geeigneten Lösungsmittel, wie beispielsweise Wasser oder einem niederen aliphatischen Alkohol, löst, eine äquivalente Menge der gewünschten Base zusetzt, für eine gute Durchmischung sorgt und nach beendeter Salzbildung das Lösungsmittel im Vakuum abdestilliert. Gegebenenfalls können die Salze nach der Isolierung umkristallisiert werden.

Pharmazeutisch verwendbare Salze sind z.B. Metallsalze, insbesondere Alkalimetall-oder Erdalkalimetallsalze, wie Natrium-, Kalium-, Magnesium-oder Calciumsalze. Andere pharmazeutisch verwendbare Salze sind beispielsweise auch leicht kristallisierende Ammoniumsalze. Letztere werden abgeleitet von Ammoniak oder organischen Aminen, z.B. Mono-, Di-oder Triniederalkyl (cycloalkyl oder -hydroxyalkyl)-aminen, Niederalkylendiaminen oder (Hydroxyniederalkyl oder Arylniederalkyl)-niederalkylammonium Basen, z.B. Methylamin, Diethylamin, Triethylamin, Dicyclohexylamin, Triethanolamin, Ethylendiamin, Tris(hydroxymethyl)-amino-methan, Benzyl-trimethylammoniumhydroxid und dergleichen.

Die Verbindungen der Formel III können, ausgehend von den literaturbekannten Verbindungen der Formel IV (EP-A 201 094) und V (C.F.H. Allen, J.B. Normington und C.V. Wilson, Can. J. Research, 11, 382 (1934)), gemäß dem folgenden Reaktionsschema und den spezifischen Angaben in den Beispielen nach üblichen und jedem Fachmann geläufigen chemischen Arbeitsmethoden synthetisiert werden.

Reaktionsschema

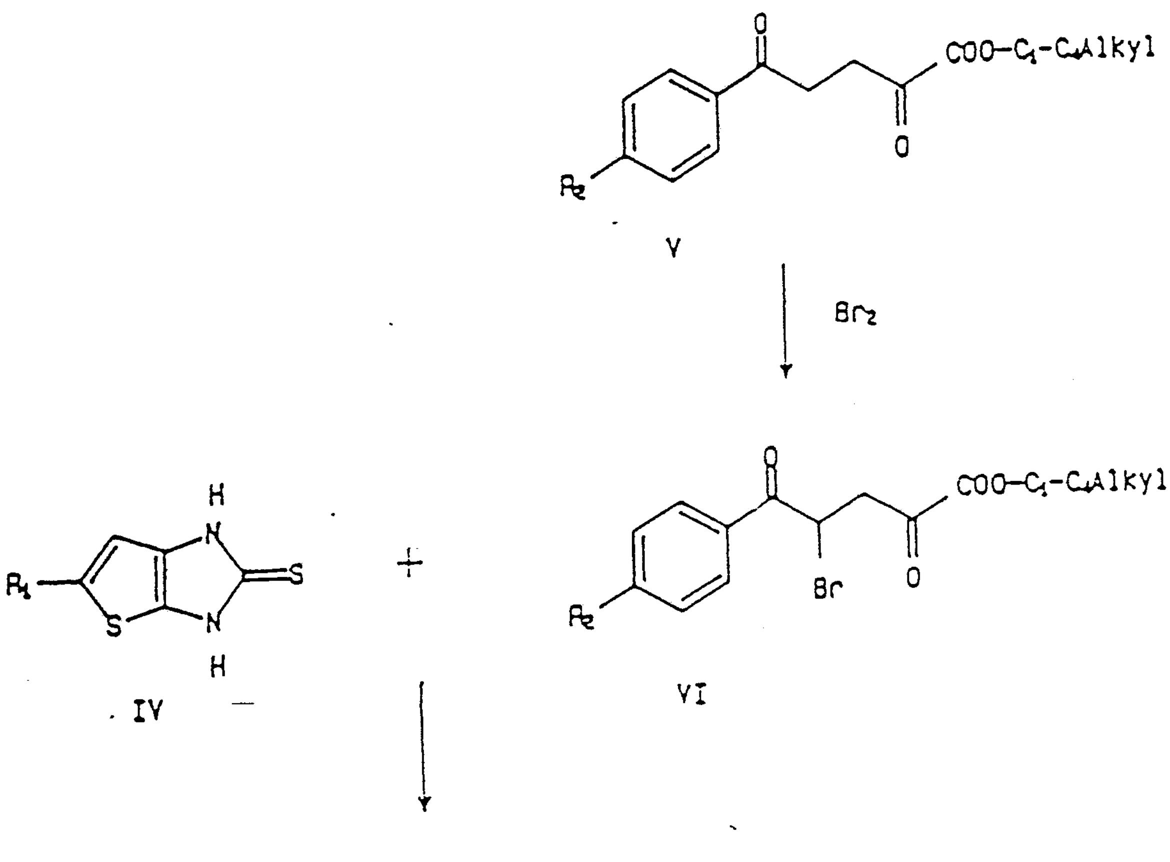

V

IV

VI

III

Die neuen Verbindungen der Formel I und ihre pharmazeutisch verwendbaren Salze zeigen in in vitro-Modellen eine hervorragende Stimulierung des Immunsystems.

Aufgrund dieser pharmakologischen Eigenschaften können die neuen Verbindungen allein oder in Mischung mit anderen Wirksubstanzen in Form üblicher galenischer Zubereitungen bei Erkrankungen, die durch ein defektes Immunsystem verursacht werden, wie z.B. Krebs oder rheumatoider Arthritis, als Medikament verwendet werden.

Die Verbindungen der Formel I sind zur Verwendung am Menschen bestimmt und können auf übliche Weise, wie beispielsweise oral oder parenteral, verabreicht werden. Vorzugsweise werden sie oral verabreicht, wobei die Tagesdosis ca. 0,1 bis 100 mg/kg Körpergewicht beträgt, vorzugsweise 0,2 bis 20 mg/kg Körpergewicht. Der behandelnde Arzt kann jedoch, abhängig vom Allgemeinzustand und dem Alter des Patienten, der entsprechenden Substanz der Formel I, der Art der Krankheit und der Art der Formulierung, auch Dosen darüber oder darunter verschreiben.

Falls die erfindungsgemäßen Substanzen zur Prophylaxe verwendet werden, bewegen sich die Dosen ungefähr im selben Rahmen wie im Behandlungsfall. Die orale Verabreichung ist auch im Fall der Prophylaxe bevorzugt.

Die Verbindungen der Formel I können allein oder in Verbindung mit anderen pharmazeutisch aktiven Substanzen verabreicht werden, wobei der Gehalt der Verbindungen der Formel I zwischen 0,1 und 99 % liegt. Im allgemeinen liegen die pharmazeutisch aktiven Verbindungen in einer Mischung mit geeigneten inerten Hilfs-und/oder Trägerstoffen oder Verdünnungsmitteln, wie z.B. pharmazeutisch unbedenklichen Lösungsmitteln, Gelatine, Gummi arabicum, Milchzucker, Stärke, Magnesiumstearat, Talk, pflanzlichen Ölen, Polyalkylenglykol, Vaseline und dergleichen vor.
Die pharmazeutischen Präparate können in fester Form, beispielsweise als Tabletten, Dragees, Suppositorien, Kapseln und dergleichen, in halbfester Form, beispielsweise als Salben oder in flüssiger Form, beispielsweise als Lösungen, Suspensionen oder Emulsionen, vorliegen. Gegebenenfalls sind sie sterilisiert und enthalten Hilfsstoffe, wie Konservierungs-, Stabilisierungs-oder Emulgiermittel, Salze zur Veränderung des osmotischen Druckes und dergleichen.

Insbesondere können pharmazeutische Präparate die erfindungsgemäßen Verbindungen in Kombination mit andern therapeutisch wertvollen Stoffen enthalten. Mit diesen können die erfindungsgemäßen Verbindungen beispielsweise zusammen mit den oben angegebenen Hils-und/oder Trägerstoffen oder Verdünnungs-mitteln zu Kombinationspräparaten formuliert werden.

Beispiel 1

5-(4-Chlorphenyl)-thieno(2',3'-4,5)imidazo(2,1-b)thiazol-6-essigsäuremethylester

11.0 g (28,9 mmol) 4-(4-Chlorphenyl)-4-oxo-3-(1H-thieno(2,3-d)-imidazol-2-yl)thio-butansäuremethylester (Formel III, $R_1$ = H, $R_2$ = Cl, $R_3$ = $CH_3$) werden in 99,0 g Phosphoroxychlorid suspendiert und 10 min zum Sieden erhitzt. Das überschüssige Phosphoroxychlorid wird abdestilliert und der Rückstand mit gesättigter Natriumhydrogencarbonatlösung neutralisiert. Diese Suspension wird einmal mit 300 ml und dreimal mit 100 ml Ethylacetat extrahiert. Die vereinten organischen Phasen werden über Natriumsulfat getrocknet, mit einem halben Teelöffel Aktivkohle gerührt, filtriert und zur Trockene eingedampft.
Der Rückstand wird dreimal aus Methanol umkristallisiert.

Ausbeute: 6,1 g gelbliche Kristalle (58,2 % d.Th.)
Fp.: 159 - 161 °C (Methanol)

$^1$H-NMR: (DMSO)
delta (ppm): 7,70 (s; 4H; Ph-H), 7,31; 7,25; 7,22; 7,16 (AB; 2H; Th-H), 3,92 (s; 2H; $-CH_2-$)

$^{13}$C-NMR: (DMSO)
delta (ppm):169,6 (s) 152,2 (s) 150,9 (s) 135,0 (s) 130,7 (d) 129,4 (d) 127,2 (s) 126,0 (s) 122,6 (s) 121,5 (d) 117,0 (s) 115,4 (d) 52,2 (q) 32,6 (t)

Das Ausgangsmaterial kann wie folgt hergestellt werden:

3-Brom-4-(4-chlorphenyl)-4-oxo-butansäuremethylester

50,0 g (0,221 mol) 4-(4-Chlorphenyl)-4-oxo-butansäuremethylester (C.F.H. Allen, J.B. Normington und C.V. Wilson, Can. J. Research, 11, 382 (1934)) werden in 250 ml Eisessig gelöst und drei Tropfen einer Lösung von Bromwasserstoff in Eisessig zugesetzt. Unter Rühren werden 35,3 g (0,221 mol) Brom so zugetropft, daß sich im Reaktionsgemisch keine merkbare Braunfärbung ausbilden kann. Nach beendeter Zugabe wird noch 15 Minuten weitergerührt und danach der Eisessig weitgehend abdestilliert.

Der Rückstand wird mit gesättigter Natriumhydrogencarbonatlösung neutralisiert und dreimal mit insgesamt 600 ml Methylenchlorid extrahiert. Die vereinten organischen Phasen werden über Natriumsulfat getrocknet, filtriert und eingedampft (67,2 g gelbes Öl).
Es wird mit Methanol angerieben und aus Methanol umkristallisiert.

Ausbeute: 65,2 g farblose Kristalle
Fp.: 48 - 49 °C (Methanol)

4-(4-Chlorphenyl)-4-oxo-3-(1H-thieno(2,3-d)imidazol-2-yl)-thiobutansäuremethylester

3,60 g (23,0 mmol) 1,3-Dihydro-thieno(2,3-d)imidazol-2-thion (hergestellt nach EP-A 201 094) und 6,40 g (20,9 mmol) 3-Brom-4-(4-chlorphenyl)-4-oxo-butansäuremethylester werden in 100 ml absolutem Methanol gelöst und eine Stunde unter Rückfluß erhitzt. Danach wird das Lösungsmittel abdestilliert und der ölige Rückstand mit 5 ml Ether angerieben. Die so erhaltenen Kristalle werden abgesaugt, mit wenig kaltem Ether digeriert und getrocknet (50 °C, 50 mbar).

Ausbeute: 9.25 g gelbe Kristalle; Hydrobromid.

Das Hydrobromid wird in 20 ml Ethylacetat suspendiert und mit gesättigter Natriumhydrogencarbonatlösung bis zur Beendigung der Gasentwicklung gerührt. Danach wird das Ethylacetat im Vakuum abdestilliert. Die Kristalle werden abgesaugt, mit kaltem, destillierten Wasser gewaschen und getrocknet (80 °C, 50 mbar).

Ausbeute: 6,45 g blaßgelbe Kristalle (81 % d.Th.)
Fp.: 170 - 172 °C (Ethanol)

Beispiel 2

5-(4-Chlorphenyl)-thieno(2',3'-4,5)imidazo(2,1-b)thiazol-6-essigsäuremethylester

6,40 g (16,8 mmol) 4-(4-Chlorphenyl)-4-oxo-3-(1H-thieno(2,3-d)imidazol-2yl)thiobutansäuremethylester werden in 30 g Polyphosphorsäure suspendiert und unter Rühren auf 80 °C erhitzt. Nach 2,5 Stunden wird das Reaktionsgemisch auf 100 ml Wasser gegossen und verrührt. Die wäßrige Lösung wird mit 100 ml Ethylacetat versetzt, auf 70 °C erhitzt und zweiphasig über HYFLO abgesaugt. Der Filterkuchen wird mehrmals mit siedendem Ethylacetat nachgewaschen. Die Phasen werden getrennt und die wäßrige Phase noch dreimal mit insgesamt 300 ml Ethylacetat extrahiert. Die vereinten organischen Phasen werden mit gesättigter Natriumhydrogencarbonatlösung extrahiert, über Natriumsulfat getrocknet, filtriert und eingedampft.
Das Gemisch der beiden isomeren Ester wird dreimal aus Methanol umkristallisiert.

Ausbeute: 1,20 g gelbliche Kristalle (19,7 % d.Th.)
Fp.: 159 - 161 °C (Methanol)
NMR: wie im Beispiel 1

Beispiel 3

7-(4-Chlorphenyl)-thieno(3',2'-4,5)imidazo(2,1-b)thiazol-6-essigsäuremethylester

Durch Eindampfen der vereinten Mutterlaugen des nach Beispiel 1 gewonnenen 5-(4-Chlorphenyl)-thieno(2',3'-4,5)imdazo(2,1-b)thiazol-6-essigsäuremethylesters werden 2,7 g Gemisch der Anellierungsisomeren gewonnen. Dieses Gemisch wird säulenchromatographisch getrennt (150 g Kieselgel 60, 0,04 - 0,063 mm, Eluens: Chloroform : Ether = 3 : 1). Die Titelverbindung wird als erste Fraktion eluiert und aus Methanol umkristallisiert.

Ausbeute: 1,10 g gelbliche Kristalle (10,5 % d.Th., bezogen auf 4-(4-Chlorphenyl)-4-oxo-3-(1H-thieno(2,3-d)-imidazol-2yl-)thiobutansäuremethylester aus Beispiel 1)

Fp.: 131 - 133 °C (Methanol)

$^{1}$H-NMR: (DMSO)
delta (ppm): 7,69 (s; 4H; Ph-H), 7,20; 7,14; 6,57; 6,51 (AB; 2H; Th-H), 3,88 (s; 2H; $-CH_2-$)

$^{13}$C-NMR: (DMSO)
delta (ppm):169,7 (s) 150,8 (s) 148,2 (s) 135,0 (s) 131,1 (d) 129,3 (d) 128,8 (s) 128,3 (s) 126,1 (s) 120,2 (d) 116,5 (s) 109,9 (d) 52,2 (q) 32,6 (t)

Beispiel 4

7-(4-Chlorphenyl)-thieno(3',2'-4,5)imidazo(2,1-b)thiazol-6-essigsäuremethylester

Durch Eindampfen der vereinten Mutterlaugen des nach Beispiel 2 gewonnenen 5-(4-Chlorphenyl)-thieno(2',3'-4,5)imidazo(2,1-b)thiazol-6-essigsäuremethylesters werden 0,60 g Gemisch der Anellierungsisomeren gewonnen. Dieses Gemisch wird säulenchromatographisch getrennnt (150 g Kieselgel 60, 0,04 - 0,063 mm, Eluens: Chloroform : Ether = 3 : 1). Die Titelverbindung wird als erste Fraktion eluiert und wird aus Methanol umkristallisiert.

Ausbeute: 0,25 g gelbliche Kristalle (4,1 % d.Th., bezogen auf 4-(4-Chlorphenyl)-4-oxo-3-(1H-thieno(2,3-d)-imidazol-2yl)-thiobutansäuremethylester aus Beispiel 2).

Fp.: 131 - 133 °C (Methanol)
NMR: wie im Beispiel 3

Beispiel 5

5-(4-Chlorphenyl)-thieno(2',3'-4,5)imidazo(2,1-b)thiazol-6-essigsäure

1,70 g (4,69 mmol) 5-(4-Chlorphenyl)-thieno(2',3'-4,5)imidazo(2,1-b)thiazol-6-essigsäuremethylester werden in einer Lösung von 270 mg (4,81 mmol) Kalium hydroxid in 8 ml Wasser und 20 ml Methanol suspendiert und 20 Minuten unter Rückfluß erhitzt. Das Lösungsmittel wird abdestilliert und der Rückstand in 15 ml Wasser aufgenommen. Die entstandene Suspension wird mit 2n Salzsäure auf pH = 1 angesäuert und nach 15 minütigem Rühren abgesaugt. Der Rückstand wird mit destilliertem Wasser gewaschen, aus Ethanol unter Verwendung von Aktivkohle umkristallisiert und getrocknet (50 °C/2 mbar).

Ausbeute: 1,60 g farblose Kristalle (98 % d.Th.)
Fp.: 239 - 240 °C (Ethanol)

$^{1}$H-NMR: (DMSO)
delta (ppm): 7,71 (s; 4H; Ph-H), 7,32; 7,26; 7,23; 7,16; (AB; 2H; Th-H), 3,82 (s; 2H; $-CH_2-$)

$^{13}$C-NMR: (DMSO)
delta (ppm): 170,6 (s) 152,1 (s) 151,2 (s) 135,0 (s) 130,7 (d) 129,3 (d) 126,8 (s) 126,2 (s) 122,5 (s) 121,4 (d) 117,9 (s) 115,5 (d) 33,1 (t)

Beispiel 6

7-(4-Chlorphenyl)-thieno(3',2'-4,5)imidazo(2,1-b)thiazol-6-essigsäure

0,30 g (0,83 mmol) 7-(4-Chlorphenyl)-thieno(3',2'-4,5)imidazo(2,1-b)thiazol-6-essigsäuremethylester werden in einer Lösung von 50 mg (0,89 mmol) Kaliumhydroxid in 5 ml Wasser und 10 ml Methanol suspendiert und unter Rückfluß erhitzt. Es entsteht eine klare Lösung. Das Lösungsmittel wird abdestilliert und und der Rückstand in 15 ml Wasser aufgenommen. Es wird mit 2n Salzsäure angesäuert (pH = 1) und nach 15 minütigem Rühren abgesaugt. Der Rückstand wird mit destilliertem Wasser gewaschen, aus Ethanol unter Verwendung von Aktivkohle umkristallisiert und getrocknet (50 °C/2 mbar).

Ausbeute: 0,28 g farblose Kristalle (97 % d.Th.)
Fp.: 235 - 238 °C (Ethanol)

$^1$H-NMR: (DMSO)
delta (ppm): 7,70 (s; 4H; Ph-H), 7,20; 7,14; 6,59; 6,53 (AB; 2H; Th-H), 3,78 (s; 2H; $-CH_2-$)

$^{13}$C-NMR: (DMSO)
delta (ppm):170,7 (s) 151,1 (s) 148,2 (s) 134,9 (s) 131,1 (d) 129,3 (d) 128,8 (s) 127,9 (s) 126,4 (s) 120,1 (d) 117.4 (s) 110,0 (d) 32,9 (t)

**Ansprüche**

1. Neue Thieno-imidazo(2,1-b)thiazol-Derivate der Formel I des Formelblattes, in der A zusammen mit den beiden Kohlenstoff-Atomen des Imidazolrings eine Gruppe der Formel IIa oder IIb des Formelblattes bildet, $R_1$ Wasserstoff, $C_1$-$C_4$ Alkyl, Halogen oder $CF_3$,
$R_2$ Wasserstoff, Halogen oder $CF_3$ und
$R_3$ Wasserstoff oder $C_1$-$C_4$ Alkyl bedeuten und, für den Fall, daß $R_3$ Wasserstoff bedeutet, ihre pharmazeutisch verwendbaren Salze.

2. Verbindungen der Formel I des Formelblattes nach Anspruch 1 und ihrer Salze, worin $R_1$ Wasserstoff, $R_2$ Chlor und $R_3$ Wasserstoff oder Methyl bedeuten.

3. 5-(4-Chlorphenyl)-thieno(2',3'-4,5)imidazo(2,1-b)thiazol-6-essigsäuremethylester.
7-(4-Chlorphenyl)-thieno(3',2'-4,5)imidazo(2,1-b)thiazol-6-essigsäuremethylester.
5-(4-Chlorphenyl)-thieno(2',3'-4,5)imidazo(2,1-b)thiazol-6-essigsäure.
7-(4-Chlorphenyl)-thieno(3',2'-4,5)imidazo(2,1-b)thiazol-6-essigsäure.

4. Verfahren zur Herstellung von Verbindungen der Formel I des Formelblattes und ihren Salzen, dadurch gekennzeichnet, daß man

a) eine Verbindung der Formel III des Formelblattes, in der $R_1$ und $R_2$ die obige Bedeutung haben und $R_3$ $C_1$-$C_4$ Alkyl bedeutet, in Gegenwart von wasserentziehenden Reagenzien zu einem Anellierungsisomerengemisch der Formel I, in der A die Gruppen der Formeln IIa und IIb bedeutet und $R_1$, $R_2$ und $R_3$ die oben genannte Bedeutung haben, cyclisiert und das Isomerengemisch trennt, worauf man

b) gegebenenfalls eine so erhaltene Verbindung der Formel I, in der $R_3$ $C_1$-$C_4$ Alkyl bedeutet, zu Verbindungen der Formel I, worin $R_3$ Wasserstoff bedeutet, alkalisch verseift und

c) erwünschtenfalls die in Verfahrensschritt b) erhaltene freie Säure der Formel I des Formelblattes mit anorganischen oder organischen Basen in ein pharmazeutisch verträgliches Salz überführt.

5. Pharmazeutische Präparate, enthaltend Verbindungen der Formel I des Formelblattes oder ein Salz davon in Kombination mit üblichen galenischen Hilfs-und/oder Trägerstoffen oder Verdünngungsmitteln.

6. Pharmazeutische Präparate, enthaltend Verbindungen der Formel I des Formelblattes oder ein Salz davon in Kombination mit anderen therapeutisch wertvollen Wirkstoffen sowie üblichen galenischen Hilfs- und/oder Trägerstoffen oder Verdünnungsmitteln.

7. Verbindungen der Formel I des Formelblattes oder ein Salz davon zur Verwendung als Wirkstoffe für Arnzeimittel zur Behandlung und Prophylaxe von Krankheiten, die durch ein defektes Immunsystem hervorgerufen werden.

Patentansprüche für folgende Vertragsstaaten : ES und GR

1. Verfahren zur Herstellung neuer Thieno-imidazo(2,1-b)thiazol-Derivate der Formel I des Formelblattes, in der A zusammen mit den beiden Kohlenstoff-Atomen des Imidazolrings eine Gruppe der Formel IIa oder IIb des Formelblattes bildet, $R_1$ Wasserstoff, $C_1$-$C_4$ Alkyl, Halogen oder $CF_3$,
$R_2$ Wasserstoff, Halogen oder $CF_3$ und
$R_3$ Wasserstoff oder $C_1$-$C_4$ Alkyl bedeuten und, für den Fall, daß $R_3$ Wasserstoff bedeutet, ihrer pharmazeutisch verwendbaren Salze dadurch gekennzeichnet, daß man

a) eine Verbindung der Formel III des Formelblattes, in der $R_1$ und $R_2$ die obige Bedeutung haben und $R_3$ $C_1$-$C_4$ Alkyl bedeutet, in Gegenwart von wasserentziehenden Reagenzien zu einem Anellierungsisomerengemisch der Formel I, in der A die Gruppen der Formeln IIa und IIb bedeutet und $R_1$, $R_2$ und $R_3$ die oben genannte Bedeutung haben, cyclisiert und das Isomerengemisch trennt, worauf man

b) gegebenenfalls eine so erhaltene Verbindung der Formel I, in der $R_3$ $C_1$-$C_4$ Alkyl bedeutet, zu Verbindungen der Formel I, worin $R_3$ Wasserstoff bedeutet, alkalisch verseift und

c) erwünschtenfalls die in Verfahrensschritt b) erhaltene freie Säure der Formel I des Formelblattes mit anorganischen oder organischen Basen in ein pharmazeutisch verträgliches Salz überführt.

2. Verfahren nach Anspruch 1, dadurch gekennzeichnet, daß man die Cyclisierung in Stufe a) mit Phosphoroxychlorid oder Polyphosphorsäure bei 60 - 110°C für 10 Minuten bis 4 Stunden durchführt.

3. Verfahrne nach einem der Ansprüche 1 oder 2, dadurch gekennzeichnet, daß man die Cyclisierung mit Phosphoroxychlorid bei Rückflußtemperatur durchführt.

4. Verfahren nach einem der Ansprüche 1 bis 3 zur Herstellung von isomerenreinen Verbindungen der Formel I, in der $R_3$ $C_1$-$C_4$ Alkyl bedeutet und A entweder eine Gruppe der Formel IIa oder eine Gruppe der Formel IIb darstellt, dadurch gekennzeichnet, daß man aus dem Anellierungsisomerengemisch der Formel I, in der A die Gruppen IIa und IIb darstellt, die Ester der Formel I, in der A die Gruppe IIa darstellt, durch mehrmalige Umkristallisation in Methanol isomerenrein isoliert und aus den vereinigten Mutterlaugen die Ester der Formel I, in der A die Gruppe IIb bedeutet, durch Säulenchromatographie auf Kieselgel isomerenrein isoliert.

Verfahren nach einem der Ansprüche 1 bis 4, dadurch gekennzeichnet, daß man als Elutionsmittel bei der Säulenchromatographie auf Kieselgel Mischungen aus Methylenchlorid, Chloroform, Benzol oder toluol einerseits und Ether andererseits verwendet.

Verfahren nach einem der Ansprüche 1 bis 5 zur Herstellung von Verbindungen der Formel I, in der $R_3$ die Bedeutung von Wasserstoff besitzt, dadurch gekennzeichnet, daß man die alkalische Verseifung nach Stufe b) mit Alkalihydroxidlösungen unter Zusatz eines Lösungvermittlers durchführt.

7. Verfahren nach einem der Ansprüche 1-6, dadurch gekennzeichnet, daß der Lösungsvermittler in Stufe b) Methanol oder Ethanol ist.

Formelblatt

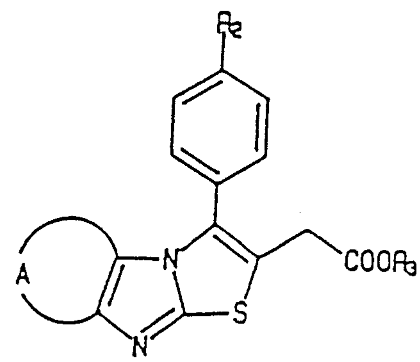

I.

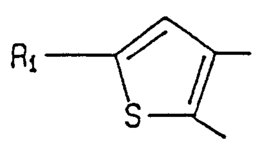

IIa

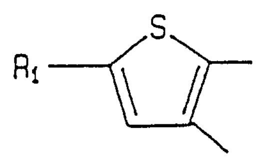

IIb

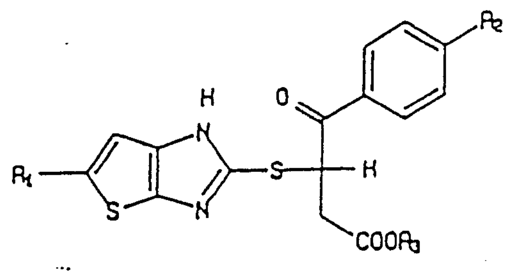

III.